# EUROPEAN PATENT SPECIFICATION

(11) **EP 2 278 880 B1**
(45) Date of publication and mention of the grant of the patent: **10.10.2012**
(21) Application number: 09729838.4
(22) Date of filing: 09.04.2009
(51) Int. Cl.: A01N 45/00, A61K 31/56

(54) **STEROID TREATMENT FOR HOT FLASHES**
STEROIDBEHANDLUNG FÜR HITZEWALLUNGEN
TRAITEMENT DES BOUFFÉES DE CHALEUR AVEC DES STÉROÏDES

(30) Priority: 09.04.2008 US 123622
(43) Date of publication of application: 02.02.2011
(73) Proprietor: Pherin Pharmaceuticals, Inc., Los Altos, CA 94022-1456 (US)
(72) Inventor: MONTI, Louis, Mountain View California 94040 (US)
(74) Representative: Baldock, Sharon Claire
(86) International application number: PCT/US2009/040102
(87) International publication number: WO 2009/126825

(56) References cited:
- US-A- 6 057 439
- US-A- 6 140 316
- US-A1- 2001 041 699
- US-A1- 2003 220 309
- US-A1- 2004 209 853
- US-A1- 2006 222 721
- US-A1- 2008 293 683
- GRINNEL ET AL: "Oestrogen protection against Acute digitalis otxiciy in dogs", NATURE, no. 4781, 17 June 1961 (1961-06-17), pages 1117-1118, XP002645781,

## Description

### BACKGROUND OF THE INVENTION

### Field of the Invention

Embodiments of the invention generally relate to the treatment of hot flashes, and more specifically relate to the treatment of hot flashes by vomeronasally administering a steroid agent to individuals.

### Description of the Related Art

### Hot Flashes

A hot flash is a momentary sensation of heat that may be accompanied by a red, flushed face and sweating by a female or male individual. The cause of hot flashes may be related to changes in circulation. Hot flashes occur when the blood vessels near the surface of the skin dilate to cool, which produces a red, flushed look to the face. An individual may also perspire to cool down the body. In addition, some individuals experience a rapid heart rate or chills. Hot flashes accompanied with sweating can also occur at night. These are called night sweats and may interfere with sleep. A hot flush is a hot flash plus a visual appearance of redness in the face and neck of the individual.

Hot flashes may be the most common symptom of menopause. Although the appearance of hot flashes coincides with estrogen withdrawal, this does not entirely explain the phenomenon because estrogen levels do not differ between symptomatic and asymptomatic women. Luteinizing hormone ("LH") pulses do not produce hot flashes, nor do changes in endogenous opiates. Recent studies suggest that hot flashes are triggered by small elevations in core body temperature ("T_{c}") acting within a reduced thermoneutral zone in symptomatic postmenopausal women. This narrowing may be due to elevated central noradrenergic activation, a contention supported by observations that clonidine and some relaxation procedures ameliorate hot flashes. Because hot flashes may be triggered by T_{c} elevations, procedures to reduce T_{c}, such as lowering ambient temperature, are beneficial. Estrogen ameliorates hot flashes by increasing the T_{c} sweating threshold, although the underlying mechanism is not known. Recent studies suggest that some sleep disturbances may be due to hot flashes while an individual is asleep.

Several health studies have shown that 75% of women surveyed reported having hot flashes in the period between perimenopause and postmenopause, with duration of symptoms for an average of 4 years. Hot flashes usually last from about 1 minute to about 5 minutes, with a small percentage persisting for about 6 minutes or longer. The experience of a hot flash is usually described as sensations of intense heat, sweating, flushing, chills, and clamminess. Sweating is reported most frequently in the face, neck, and chest, but rarely caudally.

Peripheral vasodilation, demonstrated by increased skin temperature and blood flow, occurs during hot flashes in many body areas. Skin temperatures in the digits, cheek, forehead, upper arm, chest, abdomen, back, calf, and thigh increase during hot flashes. Also, blood flow in the finger, hand, calf, and forearm may increase during hot flashes. These changes typically occur within the first few seconds of the reported onset of the hot flash. Sweating and skin conductance (electrical measure of sweating) also increase during hot flashes.

The change in the tone of the autonomic nervous system is responsible for the initiation of hot flashes. Norepinephrine ("NE") released during an increase of the sympathetic tone (or decreased parasympathetic tone) of the autonomic nervous system plays a major role in thermoregulation, acting, in part, through α₂-adrenergic receptors. When injected into the preoptic hypothalamus of laboratory animals, NE causes peripheral vasodilation and heat loss, followed by decline in body temperature. Furthermore, gonadal steroids modulate central NE activity. This theory is supported by clinical studies showing that the selective α₂-adrenergic agonist clonidine significantly reduces hot flash frequency.

Increased shivering, induced by increased skeletal muscle tone contributes to the production of body heat. The threshold for shivering, measured using the electromyogram (EMG) of skeletal muscles is lower in symptomatic postmenopausal women than in asymptomatic postmenopausal women. Some relaxation procedures that result in decreased skeletal muscle tone can help decrease hot flashes.

Analysis of the circadian rhythm of hot flashes (fit to a 24-hour period sine wave) demonstrated a circadian rhythm (p < 0.02) of hot flashes with a peak around 6:30 p.m., lagging the circadian rhythm of T_{c} in symptomatic individuals by approximately 3 hours. The majority of hot flashes were preceded by elevations in T_{c}, a statistically significant effect (p < 0.05). Hot flashes began at significantly (p < 0.02) higher levels of T_{c} (36.82 ± 0.04°C) compared with all non-flash periods (36.70 ± 0.005°C). These data are consistent with the hypothesis that elevated T_{c} serves as part of the hot flash triggering mechanism.

Since hot flashes occur in most women with the estrogen withdrawal at natural or surgical menopause, there is little doubt that estrogens are involved in their initiation. This is supported by the fact that estrogen administration nearly eliminates hot flashes. However, estrogen withdrawal alone does not explain the etiology of this symptom because there are no correlations between hot flash occurrence and plasma, urinary, or vaginal levels of estrogens, nor are there differences in plasma levels between symptomatic and asymptomatic women. Moreover, clonidine reduces hot flash frequency without changing circulating estrogen levels, and prepubertal girls have low estrogen levels but no hot flashes. Thus, estrogen withdrawal is necessary but not sufficient to explain the occurrence of hot flashes. There is temporal correspondence between pituitary pulsation of LH and temperature changes. However, LH pulses are not the basis for hot flashes.

Therefore, increased sympathoadrenergic tone (or decreased parasympathetic tone), increased body temperature, increased electrodermal activity, and increased skeletal muscle tone are conducive to hot flashes in symptomatic menopausal women.

Men may also experience hot flashes when deprived of androgens through actual castration or chemical castration. Actual castration may be the accidental or surgical loss of at least one testicle or both testicles - such as in the treatment of testicular cancer, prostate cancer, or metastatic prostate cancer. Male individuals may receive actual castration during the course of a gender transformation procedure.

Chemical castration may be induced by the administration of antiandrogen compounds or luteinizing hormone releasing hormone antagonist compounds. Chemical castration may occur during the treatment of prostate cancer or metastatic prostate cancer, but occasionally for other medical conditions. In some examples, chemical castration may be used to voluntarily decrease sexual capability of male individuals, such as in the treatment of certain sexual offenders.

### Past Treatments for Hot Flashes

Estrogen replacement therapy. The lowered estrogen levels during menopause are treated by administering 17β-estradiol systemically using oral dosage forms, nasal sprays, and lately low-dose transdermal administration using a patch. However, estrogen replacement therapy is reported to increase the risk for breast cancer, coronary heart disease ("CHD"), thromboembolism, stroke, and dementia when administered with progesterone, and increase the risk of stroke with no reduction of CHD risk when administered alone. In light of the altered risk-benefit ratios for these treatments, they are now being given at lower doses.

Several recent studies report efficacy for certain antidepressants in the treatment of hot flashes. Paroxetine, a selective serotonin-reuptake inhibitor ("SSRI") was shown to decrease hot flash composite scores by 62% (12.5 mg/day) and 65% (25.0 mg/day) in 165 women reporting 2-3 hot flashes/day. The placebo response rate was 37.8%. Fluoxetine is another SSRI used to treat hot flashes. In a study of 81 breast cancer survivors, a crossover analysis showed a reduction in hot flash frequency of about 20% over the placebo condition. Venlafaxine, a serotonin-norepinephrine reuptake inhibitor, has also shown efficacy in treating hot flashes. In a study of 229 women, venlafaxine reduced hot flash scores by 60% from baseline at 75 mg/day and 150 mg/day and 37% at 37.5 mg/day compared with 27% for placebo. Side effects of these antidepressants include nausea, dry mouth, somnolence, decreased appetite, and insomnia. Besides the side effects and the slow onset of action, antidepressants require several weeks of sustained administration before achieving therapeutic effects.

Clonidine ameliorates hot flashes by increasing the T_{c} sweating threshold. Two small placebo-controlled studies found that oral clonidine reduced hot flash frequency by 46% and transdermal clonidine reduced it by 80%. Two larger studies of breast cancer survivors receiving tamoxifen showed smaller, but significant reductions in hot flash frequency for oral and transdermal clonidine compared with placebo. However, clonidine has a slow onset of action and side effects including hypotension, dry mouth, and sedation.

Gabapentin is an anticonvulsant that binds to the α₂δ subunit of a voltage-gated calcium channel, which was fortuitously found to ameliorate hot flashes in some patients. A controlled study of 59 women found a reduction of hot flash frequency of 45% verses 29% for placebo. Side effects of gabapentin include dizziness and peripheral edema.

Isoflavones or phytoestrogens possess estrogenic properties and are found in soy products and red clover. Black cohosh is another plant-derived substance used to treat hot flashes. A recent review of 22 controlled studies, 12 studies on soy and 10 studies on other botanical compounds, found no consistent improvement of hot flashes relative to placebo.

Non-pharmaceutical treatments include procedures to reduce T_{c} and ambient temperature, such as dressing in layers and using fans or air conditioning, weight loss, smoking cessation, and relaxation procedures.

### The Vomeronasal Organ and Vomeropherins

The vomeronasal organ ("VNO"; also known as "Jacobson's organ") is a bilateral chemosensory organ found in most vertebrates including humans. In mammals, this organ may be accessed through the nostrils (as a pair of blind tubular diverticula found at the inferior margin of the nasal septum), and has been associated with pheromone reception in most species. The axons of the neuroepithelia of the vomeronasal organ, located supra palatinal, form the vomeronasal nerve and have direct input to the hypothalamus and limbic amygdala of the brain. The distal axons of the terminals nerve neurons may also serve as chemosensory receptors in the VNO. This nerve has direct synaptic connection with the hypothalamus.

Human pheromones delivered to the vomeronasal organ area bind to local receptors and trigger nerve signals that reach the brain inducing physiological and behavioral changes. Synthetic analogs of human pheromones called vomeropherins, which are substances that can bind to receptors in the VNO, may induce robust physiological, pharmacological, and behavioral changes when delivered airborne to the VNO. This information is supported by several studies in human volunteers using functional magnetic resonance imaging and positron emission tomography, showing that vomeropherins selectively activate the brain areas (hypothalamus, limbic system, cingulate gyrus, anterior thalamus, and prefrontal cortex) where their physiological, pharmacological and behavioral effects are integrated.

### SUMMARY OF THE INVENTION

Embodiments of the invention relate to methods for treating individuals suffering from hot flashes by vomeronasally administering a therapeutically effective dosage of a steroid agent. The hot flashes may be a result of postmenopause or castration suffered by the individual. In many embodiments, the method for treating individuals suffering with hot flashes is provided by vomeronasally administering a steroid agent containing an estrene compound, such as 16α,17a-epoxy-10β-hydroxyestr-4-en-3-one. In other embodiments, pharmaceutical compositions containing the steroid agent may be used to treat individuals suffering with hot flashes. Embodiments include methods for treating female or male individuals suffering from hot flashes due to postmenopause or castration by vomeronasally administering the steroid agent containing 16α,17α-epoxy-10β-hydroxyestr-4-en-3-one to levitate the hot flashes.

In one embodiment, a method for treating a male castrate suffering from hot flashes is provided which includes vomeronasally administering a therapeutically effective dosage of a steroid agent containing 16α,17α-epoxy-10β-hydroxyestr-4-en-3-one to the male castrate. The steroid agent may be administered to the male castrate at an onset of a hot flash, or alternatively, may be administered to the male castrate on a daily schedule. In some examples, the steroid agent may be administered from 2 to 8 times per day during the daily schedule, and in other examples, may be administered from 3 to 5 times per day, such as 4 times per day during the daily schedule. In some embodiments, the therapeutically effective dosage of the steroid agent contains 16α,17α-epoxy-10β-hydroxyestr-4-en-3-one at a concentration or an amount within a range from about 100 ng (nanogram) to about 4,000 ng, preferably, from about 200 ng to about 3,000 ng, more preferably, from about 400 ng to about 1,600 ng, for example, about 800 ng.

In some embodiments, the steroid agent containing 16α,17α-epoxy-10β-hydroxyestr-4-en-3-one may be topically administered to a vomeronasal organ of the male castrate. In one embodiment, the vomeronasal organ may be exposed to a nasal spray or a nasal powder spray containing the steroid agent during the administration. The nasal spray contains the steroid agent and water, and may further contain at least one excipient. The excipient may be propylene glycol, ethanol, or mixtures thereof. In another example, the nasal powder spray may contain aerosol particulate of the steroid agent. In other examples, the vomeronasal organ may be exposed to a cream, a gel, or an ointment containing the steroid agent during the administration. The steroid agent may further be coated with a time-release agent, a slow-release agent, or a controlled-release agent (e.g., pH or temperature dependent).

In some embodiments, the male castrate has actual castration, such that at least one testicle has been removed from the male castrate during a surgical castration or an accidental castration. Usually, the male castrate has had both testicles surgically or accidentally removed during the actual castration. In one embodiment, the male castrate has been administered a cancer treatment, that is, the male castrate may currently be a cancer patient or may be a former caner patient. The cancer treatment may have been for testicular cancer, prostate cancer, or metastatic prostate cancer and the actual castration was administered during the cancer treatment. In another embodiment, the male castrate may be a transsexual and the castration was administered during a surgical gender transition or sex change procedure.

In other embodiments, the male castrate has chemical castration, which may be a temporary castration or a permanent castration. In some examples, the male castrate may have been administered at least one antiandrogen compound during a treatment. The antiandrogen compound may be bicalutamide, cyproterone, flutamide, nilutamide, derivatives thereof, salts thereof, or combinations thereof. In other examples, the male castrate may have been administered at least one luteinizing hormone releasing hormone antagonist compound during the treatment. The luteinizing hormone releasing hormone antagonist compound may be buserelin, goserelin, leuprolide, triptorelin, derivatives thereof, salts thereof, or combinations thereof. In other examples, the chemically castrated man may have been administered a cancer treatment, such as for prostate cancer, metastatic prostate cancer, or brain cancer.

In one example, the male castrate may be temporally castrated during or subsequent the cancer treatment. Alternatively, the male castrate may be permanently castrated during or subsequent the cancer treatment. In some instances, the male castrate may have been chemically castrated during a treatment to reduce sexual capability and suffers hot flashes which may be treated by administering the steroid agent containing 16α,17α-epoxy-10β-hydroxyestr-4-en-3-one.

In other embodiments, the method for treating an individual suffering from hot flashes is provided which includes administering a therapeutically effectively amount of a steroid agent within a nasal passage of the individual, wherein the steroid agent binds specifically to receptors on the surface of nasal neuroepithelial cells of the individual to alleviate the hot flashes, and the nasal neuroepithelial cells are part of tissue other than olfactory epithelia. In a specific example, the method for treating a male castrate suffering from hot flashes is provided which includes administering a therapeutically effectively amount of a steroid agent within a nasal passage of the male castrate, wherein the steroid agent binds specifically to receptors on the surface of nasal neuroepithelial cells of the male castrate to alleviate the hot flashes.

In other embodiments, a method for treating a male individual having a testosterone level of about 50 ng/dL or less and suffering from hot flashes is provided which includes vomeronasally administering a therapeutically effective dosage of a steroid agent containing 16α,17α-epoxy-10β-hydroxyestr-4-en-3-one to the male individual. In some examples, the testosterone level of the male individual may be about 20 ng/dL or less.

The steroid agent may be administered to the male individual at an onset of a hot flash, or alternatively, may be administered to the male individual on a daily schedule. In some examples, the steroid agent may be administered from 2 to 8 limes per day during the daily schedule, and in other example, may be administered from 3 to 5 times per day, such as 4 times per day during the daily schedule. In some embodiments, the therapeutically effective dosage of the steroid agent contains 16α,17α-epoxy-10β-hydroxyestr-4-en-3-one at a concentration or an amount within a range from about 100 ng to about 4,000 ng, preferably, from about 200 ng to about 3,000 ng, more preferably, from about 400 ng to about 1,600 ng, for example, about 800 ng. In other embodiments, the male individual may be a male castrate having actual castration or chemical castration as described above.

In other embodiments, a method for treating an individual suffering from hot flashes during postmenopause is provided which includes vomeronasally administering a therapeutically effective dosage of a steroid agent containing an estrene compound (e.g., 16α,17α-epoxy-10β-hydroxyestr-4-en-3-one) to the individual. The postmenopause experienced by the individual suffering from hot flashes may be drug induced postmenopause, surgically induced postmenopause, or naturally occurring postmenopause. The individual to be treated with the steroid agent may be female or male.

In some examples, a male individual suffering from hot flashes due to a drug induced postmenopause may be treated by administering the steroid agent. In other examples, a male individual suffering from drug induced hot flashes may be treated by administering the steroid agent as described herein. The drug induced hot flashes may be drug induced from administration of an antiandrogen compound to the male individual undergoing androgen-dependent therapy.

In other examples, a female individual suffering hot flashes due to a surgically induced postmenopause may be treated by administering the steroid agent. In some examples, the female individual may have undergone surgery to remove or otherwise simply lacks completely or a portion of at least one female reproductive organ, such as a uterus, ovaries, fallopian tubes, and/or other female reproductive organs. The female individual may have been administered a surgery, such as during a hysterectomy. Such surgeries generally leave the female individual with considerable changes in her hormonal level which results in hot flashes.

### DETAILED DESCRIPTION

Embodiments of the invention relate to methods for treating individuals suffering from hot flashes by vomeronasally administering a therapeutically effective dosage of a steroid agent. The individual suffering from hot flashes include postmenopausal women and postmenopausal men, as well as male castrates and women who have experienced removal of her uterus, ovaries, fallopian tubes, and/or other female reproductive organs, such as during a hysterectomy.

In some embodiments, the method for treating individuals suffering with hot flashes is provided by vomeronasally administering a steroid agent containing an estrene compound, such as 16α,17α-epoxy-10β-hydroxyestr-4-en-3-one. In other embodiments, pharmaceutical compositions containing the steroid agent may be used to treat individuals suffering with hot flashes. Embodiments include methods for treating female or male individuals suffering from hot flashes due to postmenopause or castration by vomeronasally administering the steroid agent containing 16α,17α-epoxy-10β-hydroxyestr-4-en-3-one to levitate the hot flashes.

In one embodiment, a method for treating a male castrate or other individual suffering from hot flashes is provided which includes vomeronasally administering a therapeutically effective dosage of a steroid agent containing 16α,17α-epoxy-10β-hydroxyestr-4-en-3-one to the male castrate. The steroid agent may be administered to the male castrate or other individual at an onset of a hot flash, or alternatively, may be administered to the male castrate on a daily schedule. The steroid agent containing 16α,17α-epoxy-10β-hydroxyestr-4-en-3-one has a rapid onset of action for alleviating or preventing hot flashes, such as a time period within a range from about 30 seconds to about 3 minutes, immediately after vomeronasally administering the therapeutically effective dosage to the male castrate or other individual.

In some examples, the steroid agent may be administered to the individual from 2 to 8 times per day during the daily schedule, and in other examples, may be administered to the individual from 3 to 5 times per day, such as 4 times per day during the daily schedule. In some embodiments, the therapeutically effective dosage of the steroid agent contains 16α,17α-epoxy-10β-hydroxyestr-4-en-3-one at a concentration or an amount within a range from about 100 ng (nanogram) to about 4,000 ng, preferably, from about 200 ng to about 3,000 ng, more preferably, from about 400 ng to about 1,600 ng, for example, about 800 ng.

In some embodiments, the steroid agent containing 16α,17α-epoxy-10β-hydroxyestr-4-en-3-one may be topically administered to a vomeronasal organ of the male castrate or other individual. In one embodiment, the vomeronasal organ may be exposed to a nasal spray or a nasal powder spray containing the steroid agent during the administration. The nasal spray contains the steroid agent and water, and may further contain at least one excipient. The excipient may be propylene glycol, ethanol, or mixtures thereof. In another example, the nasal powder spray may contain aerosol particulate of the steroid agent. In other examples, the vomeronasal organ may be exposed to a cream, a gel, or an ointment containing the steroid agent during the administration. The steroid agent may further be coated with a time-release agent, a slow-release agent, or a controlled-release agent.

In some embodiments, the male castrate has actual castration, such that at least one testicle has been removed from the male castrate during a surgical castration or an accidental castration. Usually, the male castrate has had both testicles surgically or accidentally removed during the actual castration. In one embodiment, the male castrate has been administered a cancer treatment, that is, the male castrate may currently be a cancer patient or may be a former caner patient. The cancer treatment may have been for testicular cancer, prostate cancer, or metastatic prostate cancer and the castration was administered during the cancer treatment. In another embodiment, the male castrate may be a castrated transsexual and the castration was administered during a surgical gender transition or sex change procedure.

In other embodiments, the male castrate has chemical castration, which may be a temporary castration or a permanent castration. In some examples, the male castrate may have been administered at least one antiandrogen compound during a treatment. The antiandrogen compound may be bicalutamide, cyproterone, flutamide, nilutamide, derivatives thereof, salts thereof, or combinations thereof. In other examples, the male castrate may have been administered at least one luteinizing hormone releasing hormone antagonist compound during a treatment. The luteinizing hormone releasing hormone antagonist compound may be buserelin, goserelin, leuprolide, triptorelin, derivatives thereof, salts thereof, or combinations thereof. In other examples, the male castrate may have been administered a cancer treatment, such as for prostate cancer, metastatic prostate cancer, or brain cancer.

In one example, the male castrate may be temporally castrated during or subsequent the cancer treatment. Alternatively, the male castrate may be permanently castrated during or subsequent the cancer treatment. In some instances, the male castrate may have been chemically castrated during a treatment to reduce sexual capability and suffers hot flashes which may be treated by administering the steroid agent containing 16α,17α-epoxy-10β-hydroxyestr-4-en-3-one

In other embodiments, the method for treating an individual suffering from hot flashes is provided which includes administering a therapeutically effectively amount of a steroid agent within a nasal passage of the individual, wherein the steroid agent binds specifically to receptors on the surface of nasal neuroepithelial cells of the individual to alleviate the hot flashes, and the nasal neuroepithelial cells are part of tissue other than olfactory epithelia. In a specific example, the method for treating a male castrate suffering from hot flashes is provided which includes administering a therapeutically effectively amount of a steroid agent within a nasal passage of the male castrate, wherein the steroid agent binds specifically to receptors on the surface of nasal neuroepithelial cells of the male castrate to alleviate the hot flashes.

In other embodiments, a method for treating a male individual having a testosterone level of about 50 ng/dL or less and suffering from hot flashes is provided which includes vomeronasally administering a therapeutically effective dosage of a steroid agent containing 16α,17α-epoxy-10β-hydroxyestr-4-en-3-one to the male individual. In some examples, the testosterone level of the male individual may be about 20 ng/dL or less.

The steroid agent may be administered to the male individual at an onset of a hot flash, or alternatively, may be administered to the male individual on a daily schedule. In some examples, the steroid agent may be administered from 2 to 8 times per day during the daily schedule, and in other example, may be administered from 3 to 5 times per day, such as 4 times per day during the daily schedule. In some embodiments, the therapeutically effective dosage of the steroid agent contains 16α,17α-epoxy-10β-hydroxyestr-4-en-3-one at a concentration within a range from about 100 ng to about 4,000 ng, preferably, from about 200 ng to about 3,000 ng, more preferably, from about 400 ng to about 1,600 ng, for example, about 800 ng. In other embodiments, the male individual may be a male castrate having actual castration or chemical castration as described above.

In other embodiments, a method for treating an individual suffering from hot flashes during postmenopause is provided which includes vomeronasally administering a therapeutically effective dosage of a steroid agent containing 16α,17α-epoxy-10β-hydroxyestr-4-en-3-one to the individual. The postmenopause experienced by the individual suffering from hot flashes may be drug induced postmenopause, surgically induced postmenopause, or naturally occurring postmenopause. The individual to be treated with the steroid agent may be female or male.

In some examples, a male individual suffering from hot flashes due to a drug induced postmenopause may be treated by administering the steroid agent. In other examples, a male individual suffering from drug induced hot flashes may be treated by administering the steroid agent as described herein. The drug induced hot flashes may be drug induced from administration of an antiandrogen compound to the male individual undergoing androgen-dependent therapy.

In other examples, a female individual suffering hot flashes due to a surgically induced postmenopause may be treated by administering the steroid agent. In some examples, the female individual may have undergone surgery to remove her uterus, ovaries, fallopian tubes, and/or other female reproductive organs, such as during a hysterectomy. Such surgeries generally leave the female individual with considerable changes in her hormonal level which results in hot flashes.

In some embodiments, the steroid agent containing 16α,17α-epoxy-10β-hydroxyestr-4-en-3-one may be administered at a frequency correlated to a population circadian rhythm of hot flashes. In other embodiments, the steroid agent containing 16α,17α-epoxy-10β-hydroxyestr-4-en-3-one may be administered at a frequency correlated to a circadian rhythm of hot flashes of the male castrate.

"Hot flash" or "hot flashes" includes the momentary sensation of heat, optionally accompanied by flushing and sweating, also optionally accompanied by tachycardia and chills, as experienced in postmenopausal individuals and male castrates. The term also includes "hot flushes" and "night sweats".

A "perimenopausal woman" is a post-pubertal woman who has experienced partial, but not yet complete, cessation of menses. A "postmenopausal woman" is a post-pubertal woman who has experienced complete cessation of menses. A "menopausal woman" includes both a perimenopausal woman and a postmenopausal woman. The menopause in these women may be either natural (such as with age), surgical (such as by removal of both ovaries), or induced by chemical treatment (such as by treatment with estrogen antagonists, e.g., fulvestrant, raloxifene, tamoxifen, and toremifine).

A "castrate", "male castrate", or "castrated man" is a post-pubertal man who has experienced either actual castration or chemical castration. Chemical castration may include administering at least one antiandrogen compound, such as bicalutamide, cyproterone, flutamide, nilutamide, salts thereof, derivatives thereof, or combinations thereof. Chemical castration may also include administering at least one luteinizing hormone releasing hormone antagonist compound, such as buserelin, goserelin, leuprolide, triptorelin, salts thereof, derivatives thereof, or combinations thereof.

Generally, a normal adult male will have a testosterone level or concentration of greater than 50 ng/dL. However, a male castrate, or other male individuals having a low testosterone concentration, may have a testosterone level or concentration of about 50 ng/dL or less, for example, about 35 ng/dL or less, about 20 ng/dL or less, or even about 10 ng/dL or less. Male castrates, postmenopausal men, and other male individuals having these low testosterone concentrations frequently experience hot flashes. Therefore, in one embodiment herein, male individuals having a testosterone level or concentration of about 50 ng/dL or less and suffering from hot flashes may be treated by vomeronasally administering a therapeutically effective dosage of a steroid agent containing 16α,17α-epoxy-10β-hydroxyestr-4-en-3-one.

"Vomeronasal administration" or "vomeronasally administering" is administration to the human vomeronasal organ. In a clinical setting, this may be accomplished by the use of a probe specifically designed to administer the steroid agent essentially solely to the vomeronasal organ. A probe useful for vomeronasally administering a steroid agent is described in commonly assigned U.S. Pat. No. 5,303,703, which is incorporated herein by reference. More generally, however, vomeronasal administration includes intranasal administration in a manner that desirably directs the steroid agent generally towards the vomeronasal organ. In one example, a probe may be used to vomeronasally administer the steroid agent containing an estrene compound, such as 16α,17α-epoxy-10β-hydroxyestr-4-en-3-one for alleviating hot flashes.

A "therapeutically effective amount" is the amount of a steroid agent that, when administered to the vomeronasal organ of an individual suffering from hot flashes, is sufficient to effect treatment for the hot flashes. In one embodiment, therapeutically effective amount of the steroid agent containing an estrene compound, such as 16α,17α-epoxy-10β-hydroxyestr-4-en-3-one, for alleviating hot flashes.

"Treating" or "treatment" of hot flashes includes one or more of:
(1) inhibiting the occurrence of hot flashes,
(2) relieving hot flashes when they occur, and
(3) palliating the symptoms of hot flashes.

The preparation of the estrene compound 16α,17α-epoxy-10β-hydroxyestr-4-en-3-one is described in commonly assigned U.S. Pat. No. 6,057,439. The steroid compound 16α,17α-epoxy-10β-hydroxyestr-4-en-3-one has the chemical structure of:

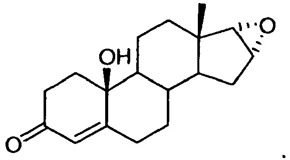

Acute and multidose (28 days) toxicity studies in rats, mice, rabbits, and dogs with single (up to 100 µg/rat, 400 µg/rabbit, 600 µg/dog) and repeated (up to 50 µg/rat/day, 300 µg/dog/day) intranasal doses and single (up to 2.5 mg/kg in rats, mice, rabbits) and repeated (up to 2.5 mg/kg/day in rats and rabbits) intravenous doses of 16α,17α-epoxy-10β-hydroxyestr-4-en-3-one demonstrated that the compound was well tolerated in all species tested, with no deaths or adverse clinical signs or effects on laboratory or pathology parameters observed.

Genotoxicity tests revealed no evidence of mutagenic or clastogenic potential of the compound when examined in the Ames reverse mutation assay and the *in vivo* bone marrow micronucleus test. Reproductive toxicity studies in pregnant rabbits revealed no adverse effects on maternal or litter parameters attributable to the compound at intravenous doses up to 2.5 mg/kg/day administered during the period of organogenesis. Preclinical pharmacokinetic studies with the compound demonstrated very low systemic exposure when the compound was administered by repeated or singe escalating intranasal doses up to 100 µg/rat, 400 µg/rabbit, 600 µg/dog. When given to rats, rabbits, or dogs in single repeated intravenous doses up to 2.5 mg/kg, plasma concentrations of the compound generally were dose-proportional and decreased rapidly.

In a group of 14 women of reproductive age, intranasal administration of a nasal spray containing 500 ng of 16α,17α-epoxy-10β-hydroxyestr-4-en-3-one per administration (administration by a Valois intranasal spray pump of 50 µL of an aqueous solution of 10 µg/mL 16α,17α-epoxy-10β-hydroxyestr-4-en-3-one with 2% propylene glycol and 2% ethanol) caused statistically significant decreases in respiratory frequency, electromyographic activity, electrodermal activity (skin conductance), and core body temperature; and statistically significant increases in vagal tone; and a not statistically significant increase in cardiac frequency compared to the vehicle.

In another test also in women of reproductive age, intranasal delivery of a nasal spray containing 1,600 ng of 16α,17α-epoxy-10β-hydroxyestr-4-en-3-one (one administration per nostril by a Valois intranasal spray pump of 50 µL of an aqueous solution of 16 µg/mL 16α,17α-epoxy-10β-hydroxyestr-4-en-3-one with 2% propylene glycol and 2% ethanol) caused a rapid (0.5 to 4 minutes latency) decrease in core body temperature of 1 ± 0.23°C that persisted for 9 ± 2.5 minutes. It also reduced the tone of the sympathoadrenergic system (as assessed by measuring physiologic sinus arrhythmia) within about 5 minutes of administration, and the effect persisted a period within a range from about 15 minutes to about 20 minutes. These results, including the lowering of core body temperature, demonstrate the efficacy of the vomeronasal administration of 16α,17α-epoxy-10β-hydroxyestr-4-en-3-one in the treatment of hot flashes.

### Formulation and administration

The steroid agent containing 16α,17α-epoxy-10β-hydroxyestr-4-en-3-one may be administered vomeronasally by a suitable route. Routes of administration include, but are not limited to, topical applications (e.g., of a dermal or preferably an intranasal cream or gel), nasal spray, nasal powder spray, and the like. Pharmaceutical formulations generally will be formulations designed to administer the drug across mucosal membranes or transdermal formulations.

Suitable delivery devices for these formulations are the metered-dose nasal spray pumps in common use for intranasal delivery of steroids for allergies and asthma, and the LHRH antagonist nafarelin for endometriosis. Such pumps are made by a number of manufacturers including Valois Pharma, Corporation. Liquid volumes are provided so that the formulation is efficiently delivered without an excess either flowing back into the nasal sinuses or dripping from the nose, and a volume of 50 µL has been found convenient, though greater or lesser volumes will also be satisfactory.

In one embodiment, a therapeutically effective dosage of a steroid agent containing 16α,17α-epoxy-10β-hydroxyestr-4-en-3-one may be from about 100 ng to about 4,000 ng, preferably, from about 200 ng to about 3,000 ng, more preferably, from about 400 ng to about 1,600 ng, for example, about 800 ng. In another embodiment, a pharmaceutical composition contains the therapeutically effective dosage of the steroid agent containing 16α,17α-epoxy-10β-hydroxyestr-4-en-3-one. The pharmaceutical composition may contain a concentration of the steroid agent within a range from about 100 ng to about 4,000 ng, preferably, from about 200 ng to about 3,000 ng, more preferably, from about 400 ng to about 1,600 ng, for example, about 800 ng. The pharmaceutical composition may be a nasal spray formulation, a nasal powder spray formulation, a cream, a gel, an ointment, a suspension, or a solution.

In one embodiment, the vomeronasal organ may be exposed to a nasal spray or a nasal powder spray containing the steroid agent during the administration to prevent or alleviate hot flashes. In one example, a nasal spray formulation may contain the steroid agent (e.g., containing 16α,17α-epoxy-10β-hydroxyestr-4-en-3-one) and water. In another example, the nasal spray formulation may further contain at least one pharmaceutically acceptable excipient. The excipient may be a glycol ether, an alcohol, or a mixture of a glycol ether and an alcohol. For example, the excipient may be propylene glycol, ethanol, or mixtures thereof. The liquid mixture of the nasal spray formulation may have a volumetric concentration of excipient within a range from about 1% to about 10%, preferably, from about 2% to about 6%, and more preferably, from about 3% to about 5%, for example, about 4%. The liquid mixture of the nasal spray formulation may have a volumetric concentration of propylene glycol within a range from about 0.5% to about 5%, preferably, from about 1% to about 3%, and more preferably, from about 1.5% to about 2.5%, for example, about 2%, and a volumetric concentration of ethanol within a range from about 0.5% to about 5%, preferably, from about 1% to about 3%, and more preferably, from about 1.5% to about 2.5%, for example, about 2%. In one example, the nasal spray contains the steroid agent within a liquid mixture containing by volume about 96% water, about 2% propylene glycol and about 2% ethanol. The nasal spray formulation may also contain a preservative, such as benzalkonium chloride. The liquid mixture of the nasal spray formulation may have a weight/volume concentration of the preservative within a range from about 0.1% to about 0.001%, for example, about 0.01%. The steroid agent may be dissolved or suspended within the liquid mixture.

In another example, the nasal powder spray may contain aerosol particulate of the steroid agent (e.g., containing 16α,17α-epoxy-10β-hydroxyestr-4-en-3-one). The aerosol particulate of the steroid agent may be administered by a pressurized gas supply, such as nitrous oxide, nitrogen, carbon dioxide, various hydrofluorocarbons, or mixtures thereof.

In other examples, the vomeronasal organ may be exposed to a cream, a gel, or an ointment containing the steroid agent (e.g., containing 16α,17α-epoxy-10β-hydroxyestr-4-en-3-one) during the administration to prevent or alleviate hot flashes. The steroid agent may further be coated with a time-release agent, a slow-release agent, or a controlled-release agent, such as by releasing the steroid agent at a particular pH or temperature.

It is expected that not more than a few percent of this dose will actually reach the vomeronasal organ, so therapeutically effective amounts when administered essentially solely to the vomeronasal organ will be perhaps 20-fold lower. These doses, both intranasal and direct vomeronasal, are well below any level that would cause a systemic effect other than those mediated through the vomeronasal organ.

Because of the rapid onset of vomeronasally administered 16α,17α-epoxy-10β-hydroxyestr-4-en-3-one, it is expected that the compound may be administered as needed, for example immediately an individual senses the onset of a hot flash, to relieve and palliate the symptoms of that hot flash. It is also expected that the compound may be administered in a scheduled fashion throughout the day, such as from about 2 times/day to 8 times/day, for example, from about 3 times/day to 5 times/day, such as 4 times/day, to prevent the occurrence of hot flashes. Such scheduled administration may be on a uniform schedule, for example at 8 a.m., noon, 4 p.m., and 8 p.m. (for 4 times/day administration), or on a nonuniform schedule where the frequency of administration is correlated with the circadian rhythm of hot flashes, either in the symptomatic population or of the individual being treated. Thus, for example, administration might be at 9 a.m., 3 p.m., 5 p.m., and 7 p.m. (again for 4 times/day) to maximize the administration at the time when the frequency of occurrence of hot flashes is greatest. Of course, even if scheduled administration is being used, it is possible to administer the compound on an as-needed basis if hot flashes are still experienced.

While the foregoing is directed to embodiments of the invention, other and further embodiments of the invention may be devised without departing from the basic scope thereof, and the scope thereof is determined by the claims that follow.

## Claims

1. A steroid agent comprising 16α,17α-epoxy-10β-hydroxyestr-4-en-3-one for use in vomeronasally treating an individual suffering from hot flashes, where the individual is a menopausal woman or a castrate man.

2. The agent of claim 1, where the individual is a menopausal woman.

3. The agent of claim 2, where the individual is a perimenopausal woman.

4. The agent of claim 2, where the individual is a postmenopausal woman.

5. The agent of claim 1, where the individual is a castrate man.

6. The agent of any one of claims 1 to 5, where the 16α,17α-epoxy-10β-hydroxyestr-4-en-3-one is administered at the onset of a hot flash.

7. The agent of any one of claims 1 to 5, where the 16α,17α-epoxy-10β-hydroxyestr-4-en-3-one is administered on a schedule throughout the day.

8. The agent of claim 7, where the 16α,17α-epoxy-10β-hydroxyestr-4-en-3-one is administered from 2 to 8 times per day.

9. The agent of claim 8, where the 16α,17α-epoxy-10β-hydroxyestr-4-en-3-one is administered from 3 to 5 times per day.

10. The agent of claim 9, where the 16α,17α-epoxy-10β-hydroxyestr-4-en-3-one is administered 4 times per day.

11. The agent of any one of claims 7 to 10, where the frequency of administration of the 16α,17α-epoxy-10β-hydroxyestr-4-en-3-one is correlated with the population circadian rhythm of hot flashes.

12. The agent of any one of claims 7 to 10, where the frequency of administration of the 16α,17α-epoxy-10β-hydroxyestr-4-en-3-one is correlated with the circadian rhythm of hot flashes for the individual being treated.

13. The agent of any one of claims 1 to 12, where the 16α,17α-epoxy-10β-hydroxyestr-4-en-3-one is administered in a nasal spray.

14. The agent of claim 13, where the nasal spray comprises an aqueous solution of 16α,7α-epoxy-10β-hydroxyestr-4-en-3-one.

15. The agent of claim 13 or 14, where the 16α,17α-epoxy-10β-hydroxyestr-4-en-3-one content of the nasal spray is 200 to 3000 nanograms per administration.

16. The agent of claim 15, where the 16α,17α-epoxy-10β-hydroxyestr-4-en-3-one content of the nasal spray is 400 to 1600 nanograms per administration.

17. The agent of claim 1, where the 16α,17α-epoxy-10β-hydroxyestr-4-en-3-one is for vomeronasally treating an individual suffering from surgically induced hot flashes or drug induced hot flashes.

## Patentansprüche

1. Steroid Wirkstoff umfassend 16α,17α-epoxy-10β-hydroxyestr-4-en-3-on zur Verwendung in vomeronasaler Behandlung eines Individuums, welches an Hitzewallungen (hot flashes) leidet, wobei das Individuum ein Frau in der Menopause oder ein kastrierter Mann ist.

2. Wirkstoff nach Anspruch 1, wobei das Individuum ein Frau in der Menopause ist.

3. Wirkstoff nach Anspruch 2, wobei das Individuum ein Frau in der Perimenopause ist.

4. Wirkstoff nach Anspruch 2, wobei das Individuum ein Frau in der Postmenopause ist.

5. Wirkstoff nach Anspruch 1, wobei das Individuum ein kastrierier Mann ist.

6. Wirkstoff nach Ansprüchen 1 bis 5, wobei das 16α,17α-epoxy-10β-hydroxyestr-4-en-3-on beim Beginn einer Hitzewallung verabreicht wird.

7. Wirkstoff nach Ansprüchen 1 bis 5, wobei das 16α,17α-epoxy-10β-hydroxyestr-4-en-3-on nach einem Zeitplan über den Tag verteilt verabreicht wird.

8. Wirkstoff nach Anspruch 7, wobei das 16α,17α-epoxy-10β-hydroxyestr-4-en-3-on 2 bis 8 mal pro Tag verabreicht wird.

9. Wirkstoff nach Anspruch 8, wobei das 16α,17α-epoxy-10β-hydroxyestr-4-en-3-on 3 bis 5 mal pro Tag verabreicht wird.

10. Wirkstoff nach Anspruch 9, wobei das 16α,17α-epoxy-10β-hydroxyestr-4-en-3-on 4 mal pro Tag verabreicht wird.

11. Wirkstoff nach Ansprüchen 7 bis 10, wobei die Frequenz der Verabreichung von dem 16α,17α-epoxy-10β-hydroxyestr-4-en-3-on mit dem Bevölkerungs-circadianem Rhythmus der Hitzewallungen kordel ist.

12. Wirkstoff nach Ansprüchen 7 bis 10, wobei die Frequenz der Verabreichung von dem 16α,17α-epoxy-10β-hydroxyostr-4-en-3-on mit dem circadianem Rhythmus der Hitzewallungen des zu behandelnden Individuums korreliert ist.

13. Wirkstoff nach Ansprüchen 1 bis 12, wobei das 16α,17α-epoxy-10β-hydroxyestr-4-en-3-on in einem Nasenspray verabreicht wird.

14. Wirkstoff nach Anspruch 13, wobei das Nasenspray eine wässrige Lösung von 16α,17α-epoxy-10β-hydroxyestr-4-en-3-on umfasst.

15. Wirkstoff nach Ansprüchen 13 oder 14, wobei der Gehabt an 16α,17α-epoxy-10β-hydroxyestr-4-on-3-on in dem Nasenspray 200 bis 3000 Nanogramm pro Verabreichung ist.

16. Wirkstoff nach Anspruch 15, wobei der Gehalt an 16α,17α-epoxy-10β-hydroxyostr-4-en-3-on in dem Nasenspray 400 bis 1600 Nanogramm pro Verabreichung ist.

17. Wirkstoff nach Anspruch 1, wobei das 16α,17α-opoxy-10β-hydroxyostr-4-en-3-on zur vomeronasalen Behandlung von einem Individuum ist, welches an chirurgisch-induzierton Hitzewallungen oder arzneimittelinduzierten Hitzewallungen leidet.

## Revendications

1. Agent stéroïdien comprenant de la 16α,17α-époxy-10β-hydroxyestr-4-ène-3-one pour une utilisation pour le traitement voméronasal d'un individu souffrant de bouffées de chaleur, ledit individu étant un sujet féminin ménopausé ou un sujet masculin castré.

2. Agent suivant la revendication 1, l'individu étant un sujet féminin ménopausé.

3. Agent suivant la revendication 2, l'individu étant un sujet féminin en périménopause.

4. Agent suivant la revendication 2, l'individu étant un sujet féminin en postménopause.

5. Agent suivant la revendication 1, l'individu étant un sujet masculin castré.

6. Agent suivant l'une quelconque des revendications 1 à 5, la 16α,17α-époxy-10β-hydroxyestr-4-ène-3-one étant administrée au début d'une bouffée de chaleur.

7. Agent suivant l'une quelconque des revendications 1 à 5, la 16α,17α-époxy-10β-hydroxyestr-4-ène-3-one étant administrée suivant un schéma tout au long de la journée.

8. Agent suivant la revendication 7, la 16α,17α-époxy-10β-hydroxyestr-4-ène-3-one étant administrée 2 à 8 fois par jour.

9. Agent suivant la revendication 8, la 16α,17α-époxy-10β-hydroxyestr-4-ène-3-one étant administrée 3 à 5 fois par jour.

10. Agent suivant la revendication 9, la 16α,17α-époxy-10β-hydroxyestr-4-ène-3-one étant administrée 4 fois par jour.

11. Agent suivant l'une quelconque des revendications 7 à 10, la fréquence d'administration de la 16α,17α-époxy-10β-hydroxyestr-4-ène-3-one étant corrélée avec le rythme circadien des bouffées de chaleur de la population.

12. Agent suivant l'une quelconque des revendications 7 à 10, la fréquence d'administration de la 16α,17α-époxy-10β-hydroxyestr-4-ène-3-one étant corrélée avec le rythme circadien des bouffées de chaleur de l'individu traité.

13. Agent suivant l'une quelconque des revendications 1 à 12, la 16α,17α-époxy-10β-hydroxyestr-4-ène-3-one étant administrée en atomisation nasale.

14. Agent suivant la revendication 13, l'atomisation nasale comprenant une solution aqueuse de 16α-17α-époxy-10β-hydroxyestr-4-ène-3-one.

15. Agent suivant la revendication 13 ou 14, la teneur en 16α,17α-époxy-10β-hydroxyestr-4-ène-3-one de l'atomisation nasale étant de 200 à 3000 nanogrammes par administration.

16. Agent suivant la revendication 15, la teneur en 16α,17α-époxy-10β-hydroxyestr-4-ène-3-one de l'atomisation nasale étant de 400 à 1600 nanogrammes par administration.

17. Agent suivant la revendication 1, la teneur en 16α,17α-époxy-10β-hydroxyestr-4-ène-3-one destinée au traitement voméronasal d'un individu souffrant de bouffées de chaleur induites chirurgicalement ou de bouffées de chaleur induites par un médicament.
